# EUROPÄISCHE PATENTANMELDUNG

(11) **EP 2 586 856 A1**
(43) Veröffentlichungstag der Anmeldung: **01.05.2013**
(21) Anmeldenummer: 12190262.1
(22) Anmeldetag: 26.10.2012
(51) Int. Cl.: C12M 1/107

(54) **Verfahren und Behälter zur Vorbehandlung organischer Stoffe einer Biogasanlage**

(30) Priorität: 28.10.2011 DE 102011085474
(71) Anmelder: Rohn, Peter, 91610 Insingen (DE)
(72) Erfinder: Rohn, Peter, 91610 Insingen (DE)
(74) Vertreter: Dreiss

(57) **Zusammenfassung**

Verfahren zum Betrieb eines Behälters (10) zur Vorbehandlung organischer Stoffe in einer Biogasanlage, es wird vorgeschlagen, dass dem Behälter (10) Verdünnungsflüssigkeit zugeführt wird, solange ein vorgegebener pH-Wert bzw. Betriebsfüllstand nicht überschritten ist.

Ferner, einen Behälter zur Vorbehandlung organischer Stoffe einer Biogasanlage umfassend eine Behältereinlaufstutzen und eine pH-Sonde, vorgeschlagen wird, dass der Behältereinlaufstutzen als 90°-Bogen ausgeführt ist, wobei eine durch den Behältereinlaufstutzen in den Behälter einströmende Verdünnungsflüssigkeit die pH-Sonde umströmt.

## Beschreibung

Die Erfindung betrifft ein Verfahren zur Vorbehandlung organischer Stoffe einer Biogasanlage, sowie einen Behälter umfassend eine Behältereinlaufstutzen und eine pH-Sonde.

Biogas entsteht durch den natürlichen Prozess des mikrobiellen Abbaus organischer Stoffe, wie Gülle, Mist oder Energiepflanzen, unter anaeroben Bedingungen. Dabei setzen Mikroorganismen die enthaltenen Kohlenhydrate, Eiweiße und Fette in die Hauptprodukte Methan und Kohlenstoffdioxid um. Der Prozess besteht aus mehreren Stufen, die jeweils von Mikroorganismen verschiedener Stoffwechseltypen durchgeführt werden.

Weil sich die Milieuanforderungen der unterschiedlichen an der Biogaserzeugung beteiligten Mikroorganismen deutlich voneinander unterscheiden, ist es bekannt, eine erste Stufe, die sogenannte Hydrolyse oder Versäuerung, in einem separaten Behälter durchzuführen und anschließend das dabei entstandene Substrat in einen zweiten Behälter, den sogenannten Fermenter umzupumpen, um dort die eigentliche Methanisierung des Substrates durchzuführen.

Die Hydrolyse unterliegt großen Prozessschwankungen, vor allem beim Nachfüllen von Rohstoffen, der sogenannten Fütterung. Hierbei werden organische Feststoffe mit flüssigen Gärresten aus dem Gärrestelager oder Nachgärer vermischt, um ein pumpfähiges Substrat zu erhalten. Weil die Gärreste über einen pH-Wert von 7,2 bis 8,2 verfügen, steigt der, für die Hydrolyse benötigte, pH-Wert von 4,5 bis 7 für mehrere Stunden auf höhere Werte an. Dadurch sinkt einerseits der Säuregehalt des Substrates wodurch die nachfolgende Fermentation nicht optimal abläuft. Andererseits werden bei erhöhten pH-Werten geringe Mengen Methan und Wasserstoff gebildet, die in dieser Prozessstufe nicht abgeschieden werden können und so für die Energieerzeugung verloren sind. Es sei denn, der Hydrolysebehälter wird in gasdichter Bauweise erstellt, wodurch deutlichere Mehrkosten, für Sicherheitseinrichtungen, Gasstrecke und vor allem für einen Feststoffdosierer, mit einer oder mehreren Förderschnecken, entstehen. Der sehr geringe Methangehalt des Hydrolysegases und die dadurch verursachten Reduzierung des Methangehaltes, des im Fermenter erzeugten Biogases, wirken sich nachteilig auf die Rentabilität des gasdichten Hydrolysebehälters aus.

Die Praxis zeigt, dass Biogasanlagen mit vorgeschalteter Hydrolyse, in nicht gasdichten Behältern, einen Methangehalt von ca. 55 bis 65 % im Biogas erreichen, der damit ca. 5 bis 15 % höher liegt als bei herkömmlichen Anlagen, ohne Hydrolyse.

Für die Auslegung und beim Bau des Hydrolysebehälters ist auf jeden Fall der für die Anlage zuständige Sachverständige für Betriebssicherheit mit einzubeziehen.

Durch das erfindungsgemäße Verfahren zum Betreiben des Behälters wird der pH-Wert innerhalb vorgebbarer Grenzen, d. h. zwischen einem Minimalwert und einem Maximalwert, bei dem die Substratversäuerung optimal ablaufen kann, konstant gehalten. Dadurch wird die Bildung von Methan bei der Vorbehandlung nahezu vermieden und die damit einhergehenden Energieverluste reduziert. Außerdem wird durch gleichmäßig gut versäuertes Substrat die Leistung des Fermenters konstant hoch gehalten.

Eine Grundidee des erfindungsgemäßen Verfahrens ist es, mittels einer frequenzgesteuerten Pumpe mit geringer Förderleistung, eine Verdünnungsflüssigkeit oder Gülle so in den Behälter einzubringen, dass der pH-Wert vorgebbare Grenzen nicht überschreitet. Dadurch werden die Prozessschwankungen reduziert. Die zuführbare Verdünnungsmenge wird zusätzlich durch einen in die Steuerung eingebbaren Betriebsfüllstand begrenzt. Dieser liegt in der Praxis ca. 1m unterhalb der Behälterdecke, da ein Reservevolumen für aufschwimmende Futterkomponenten vorgehalten werden muss.

Die Regelung des Betriebsfülllstands sollte mit kleinen Mengen an Verdünnungsflüssigkeit und relativ häufig erfolgen, um den gewünschten Betriebsfülllstand relativ schwankungsfrei zu gewährleisten. Dies wird durch die Anlagensteuerung und die zugehörige Software sichergestellt. Hierdurch wird das Behälterversäuerungsvolumen immer zu fast 100% ausgenutzt und die Rührwerksflügel können im optimalen Abstand zum gleichbleibender Füllstand eingebaut werden. Daraus ergibt sich eine weitere Verbesserung der Leistungsfähigkeit und der Wirtschaftlichkeit des Behälters.

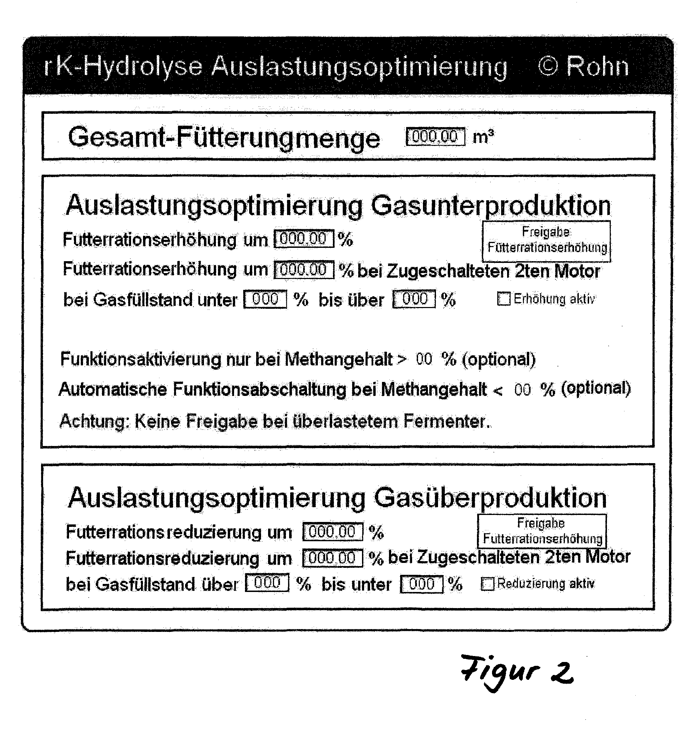

Bei einer realisierten Anlage mit einem Hydrolyse-Behälter mit 4,5m Tiefe wird der Betriebsfüllstand relativ stabil vom System bzw. der Steuerung automatisch bei 3,5m gehalten. Das Abpumpen der Fermenterfütterungsmenge die durch die Auslastungsoptimierung in Abhängigkeit vom Gasfüllstand festgelegt wird (siehe die Beschreibung der Figur 2), erfolgt z.B. 1mal pro Stunde mit 4m³. Um dies auszugleichen wird gleichzeitig z.B. 4mal pro Stunde 1m³ Verdünnungsflüssigkeit vom Durchflusszähler gemessen zugeführt; jedoch immer nur maximal so viel bis der Betriebsfüllstand erreicht ist.

Somit wird ein schwankungsfreier Füllstand gewährleistet und die Rührwerksflügel können in der optimalen Höhe eingebaut werden, so dass jederzeit ein schnelles Einrühren der Feststoffe möglich ist.

Im Praxisbetrieb wurde überraschenderweise festgestellt, dass wenn an einem Wochentag, z.B. am Sonntag, auf den Einwurf von Feststoffen verzichtet wird und der Hydrolyse nur Verdünnungsflüssigkeit zugeführt wird, die produzierte Gasmenge trotzdem ausreicht, um den mit dem Biogas betriebenen Motor eines Stromerzeugers oder -Generators nahe am oder im Volllastbereich zu betreiben. Dies bedeutet, dass die Anlage am Sonntag vollautomatisch arbeiten kann und der Betreiber sich nicht um die Anlagenfütterung kümmern muss.

In einer Versuchsanlage werden an 6 Tagen (Montag bis Samstag) pro Woche 16t Feststoffe einmal täglich in die Hydrolyse eingebracht sowie ca. 78m3 Verdünnungsflüssigkeit aus dem Nachgärer. Am siebten Tag (Sonntag) erfolgt kein Feststoffeintrag in die Hydrolyse wobei die Zufuhr von Verdünnungsflüssigkeitszufuhr in die Hydrolyse um ca. 29m3 auf etwa 107 m3 automatisch bis zum Erreichen des Betriebsfüllstandes erhöht wird, was einer Steigerung von ca. 37% entspricht.

Gleichzeitig wird vom Steuerungssystem die FermenterFütterungsmenge von normalerweise ca. 93m3 täglich um ca. 11% auf 103m3 erhöht, was gewährleistet, dass die Anlage auch am Sonntag im Volllastbetrieb von 250kw arbeitet, ohne dass Feststoffe per Radlader gefüttert werden müssen.

Da sich die Energiedichte in den 200 Kubikmetern Hydrolyse am Sonntag etwa halbiert, aber nur 11% mehr Menge in den Fermenter gefüttert wird, ergibt sich eindeutig eine höhere Ausbeute aus den Inputstoffen, die etwa 10-20% (Wochendurchschnitt) über den normal üblichen Werten anderer Anlagen liegt.

Auch die erfindungsgemäße Verteilung des in dem Hydrolysebehälter erzeugten HEB (Hydrolyseenergiebrei) mit anschließender Verteilung auf eine oder mehrere Satelliten-Biogas-Anlagen ermöglicht die Verwertung des Biogases und der dabei entstehenden Abwärme vor Ort, dort wo ein Wärmebedarf besteht, und hält die Investitionskosten niedrig.

Es hat sich weiter als vorteilhaft erwiesen, die Verdünnungsflüssigkeit thermisch vorzubehandeln, um einen höheren Abbaugrad zu erzielen.

Weitere Merkmale, Anwendungsmöglichkeiten und Vorteile der Erfindung ergeben sich aus der nachfolgenden Beschreibung von Ausführungsbeispielen der Erfindung, die in den Figuren der Zeichnung dargestellt sind. Dabei bilden alle beschriebenen oder dargestellten Merkmale für sich oder in beliebiger Kombination den Gegenstand der Erfindung, unabhängig von ihrer Zusammenfassung in den Patentansprüchen oder deren Rückbeziehung sowie unabhängig von ihrer Formulierung bzw. Darstellung in der Beschreibung bzw. in der Zeichnung.

Es zeigen:
- Figur 1: eine schematische Darstellung eines Behälters zur Vorbehandlung organischer Stoffe
- Figur 2: eine Bildschirmmaske

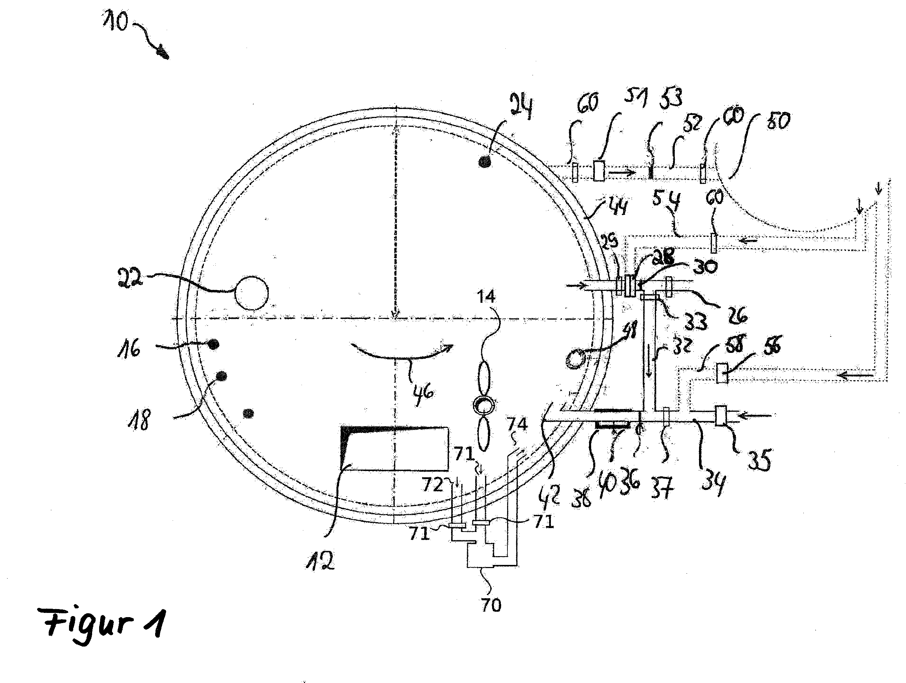

Figur 1 zeigt den erfindungsgemäßen Behälter 10 einer Biogasanlage in Draufsicht. Solche Behälter 10 sind üblicher Weise aus Beton gefertigte, zylinderförmige Hohlkörper, mit beispielsweise 8,5 m Durchmesser und 4,5 m Höhe. Ein Füllvolumen beträgt dabei 250 m³. Der Behälter 10 verfügt über eine Einfüllöffnung 12, durch die per Radlader oder Schneckenförderer Rohstoff zugeführt wird, ein Rührwerk 14, einen Füllstandsensor 16, eine Überfüllsicherung 17 und einen Temperaturfühler 18. Abhängig von der Behältergröße oder den bevorzugt eingesetzten Rohstoffen kann optional ein weiteres Rührwerk 22 eingebaut werden. Weiterhin verfügt der Behälter 10 über eine Öffnung 24 durch die das beim Prozess entstehende Gas in einen Biofilter oder zum Fermenter abgesaugt wird. Die für die Hydrolyse erforderliche Prozesstemperatur von über 20°C wird durch eine, in der Zeichnung nicht dargestellte, Wandheizung sichergestellt.

Eine Fütterungsleitung 26 verbindet den Behälter 10 mit einem Fermenter (nicht dargestellt). In der Fütterungsleitung 26 angeordnet ist eine Pumpe 28, die Substrat aus dem Behälter 10 zum Fermenter fördert und dabei im Substrat enthaltene grobe Anteile zerkleinert. Zwischen Behälter 10 und Pumpe 28 ist ein Absperrorgan, umfassend einen automatischer Schieber 29, angeordnet.

In Strömungsrichtung gesehen hinter der Pumpe 28 ist ein Durchflusszähler 30 angeordnet. Im Anschluss an den Durchflusszähler 30 zweigt eine Bypassleitung 32 von der Fütterungsleitung 26 ab. Stromabwärts hinter der Bypassleitung 32 trennt ein als automatischer Schieber 31 ausgeführtes Absperrorgan die Fütterungsleitung 26 vom Fermenter. Die Bypassleitung 32 verbindet die Fütterungsleitung 26 mit einer Zulaufleitung 34. Ein automatischer Schieber 33, angeordnet in der Bypassleitung 32, trennt die Fütterungsleitung 26 von der Zulaufleitung 34. Über die Zulaufleitung 34 fördert eine frequenzgesteuerte Pumpe 35 Verdünnungsflüssigkeit, beispielsweise flüssige Gärreste aus einem Gärrestelager oder aus einem Nachgärer (nicht dargestellt) oder Gülle, in den Behälter 10.

In der Zulaufleitung 34 ist stromabwärts, hinter der Bypassleitung 32 ein zweiter Durchflusszähler 36 angeordnet. Zwischen dem zweiten Durchflusszähler 36 und dem Behälter 10 sind optional eine thermische Aufbereitung 38 ausgeführt als Heizeinrichtung und/oder eine Belüftungseinrichtung 40 angeordnet. In der Zulaufleitung 34 ist stromaufwärts zwischen Bypassleitung 32 und frequenzgesteuerter Pumpe 35 ein automatischer Schieber 37 angeordnet.

Ein Behältereinlaufstutzen 42, angeordnet ca. 0,5 m über einem Boden des Behälters 10, ist als 90°-Bogen ausgeführt, so dass die über die Zulaufleitung 34 einströmende Verdünnungsflüssigkeit direkt an einer zylindrischen Behälterinnenwand 44 entlang geleitet wird und so eine Drehrichtung, dargestellt durch Pfeil 46, die dem Substrat im Behälter 10 durch das Rührwerk 14 und/oder das Rührwerk 22 aufgeprägt wird, unterstützt.

Stromabwärts des Behältereinlaufstutzens 42, in einer horizontalen Entfernung von ca. 1 m vom Behältereinlaufstutzen 42, ist in gleicher Höhe über dem Boden, eine pH-Sonde 48 angeordnet. Die Anordnung der pH-Sonde 48 in geringer Entfernung vom Behältereinlaufstutzen 42 ermöglicht die pH-Wert-Bestimmung auch bei kleinen Mengen an zugeführter Verdünnungsflüssigkeit, ohne dass das Rührwerk 14 anlaufen muss. Das Rührwerk 14 ist in der Nähe des Behältereinlaufstutzens 42 angeordnet.

Um eine Zerkleinerung insbesondere der (lang-)faserigen Futterkomponenten wie Gras oder strohiger Festmist, in der Hydrolyse zu erreichen, kann ein Intensivzerkleinerer 70 mit integrierter angeflanschter Pumpe an die Hydrolyse angebaut werden. Solche Intensivzerkleinerer sind am Markt erhältlich, so dass eine detaillierte Beschreibung entbehrlich ist. Es hat sich bei versuchen als vorteilhaft herausgestellt, wenn die faserigen Futterkomponenten auf eine durchschnittliche Länge von weniger als 15 mm, bevorzugt von weniger als 10 mm zerkleinert werden. Zusätzlich bewirkt der Intensivzerkleinerer auch ein "Zerfasern" der faserigen Futterkomponenten, was deren Aufschluss in der Hydrolyse erleichtert und beschleunigt.

Ein Entnahmestutzen 71 des Intensivzerkleinerers 70 sollte etwas unterhalb des Betriebsfüllstandes angebracht sein, damit sich zwischen der Schwimmdecke und dem Entnahmestutzen 71 noch Flüssigkeit befindet. Optional kann ein zweiter Entnahmestutzen 72 weiter unten am Behälter angebracht werden. Da beide Entnahmestuten 71, 72 jeweils mit einem stufenlos einstellbaren Schieber 73 ausgerüstet sind, kann eine Mischung zwischen unzerkleinerten und bereits zerkleinertem Substrat eingestellt werden, um Verstopfungen zu vermeiden.

Ein Einbringstutzen 74 sollte in Drehrichtung der in dem Behälter befindlichen Flüssigkeit mit einem ca. 45° Bogen versehen werden; dies kann auch bei den Entnahmestutzen 71, 72 in umgekehrter Richtung erfolgen, um die Rührleistung zu erhöhen.

Der Betrieb des Intensivzerkleinerers 70 sollte bevorzugt in den Rührpausen erfolgen, damit das aufschwimmende Material angesaugt werden kann. Bei längerer Laufzeit kann das Einschalten der Rührwerke 14, 22 erforderlich sein. Die Laufzeit kann auch in Abhängigkeit des PH-Werts gesteuert/geregelt werden.

Je nach eingesetzten Futterkomponenten muss der Intensivzerkleinerer 70 mehrere Stunden am Tag laufen. Ziel ist es, nach der Intensivzerkleinerung einen homogenen Brei zu erhalten, der sich nicht mehr oder zumindest kaum noch entmischt. Hierdurch kann Rührenergie eingespart werden und voraussichtlich auch ein höherer Trockensubstanzgehalt (ca. 15%) in der Hydrolyse gefahren werden, was zur Reduzierung der täglich benötigten Verdünnungsflüssigkeit führt. Optional können die eingesetzten Feststoffe bereits vor der Einbringung z.B. mit einer Spezialmühle intensiv zerkleinert werden.

Der vom Intensivzerkleinerer 70 erzeugte Hydrolyse-Energiebrei (HEB) könnte auch von einer zentralen Hydrolysestation (ZHS), die sich auf einer größeren Biogasanlage befindet, produziert und in einem Behälter zwischengelagert werden. Kleinere Satelliten-Biogas-Anlagen können dann per Tankwagen mit HEB aus dem Behälter beliefert werden.

Diese kleinen Satelliten-Biogas-Anlagen würden lediglich einen HEB-Lagerbehälter und eine Entnahmepumpe für die Fermenter-Fütterung benötigen. Auf ein eigenes Fahrsilo und einen teuren Feststoffdosierer könnte verzichtet werden. Gerade auch kleine Anlagen könnten so Investitionskosten einsparen und würden kaum Arbeitszeit für die tägliche Fütterung benötigen. Durch den schnell abbaubaren HEB könnte bis zur doppelten Leistung mit dem Fermenter relativ schnell erzeugt werden, wodurch nach dem neuen Einspeisegesetz höhere Strompreise zu erzielen sind.

Die nachgeschalteten Endlager auf der Satelliten-Biogas-Anlage könnten relativ klein gehalten werden, (nur für die eigene Güllemenge) da der Tankwagen auf dem Rückweg zur Zentrale gleich wieder Gärreste mitnehmen würde, der ja auch zum Anmaischen in der zentralen Hydrolyse benötigt wird. Da die Energiedichte des HEB je nach eingesetzten Futterkomponenten ca. bei 50% vom Silomais läge, wäre der Transport durchaus rentabel.

Die Liefermenge könnte auf einer zentralen Brückenwaage verwogen werden. Auf der belieferten Satellitenanlage könnte beim Abpumpen online der TS-Gehalt per Sonde gemessen und die angelieferten Kubikmeter können über einen Durchflusszähler erfasst werden. Der Energiegehalte des HEB kann durch die genaue Verwiegung und Erfassung der täglichen Futtermengen pro Komponente, die in der zentralen Hydrolysestation eingebracht wurden, errechnet werden.

Mit der Kostenrechnung würde der Endkunde hiervon einen Logbuchauszug erhalten, sowie einen Wiegeschein und den bestimmten Trockensubstanzgehalt der Lieferung. Die Satelliten-Biogas-Anlagen können dort gebaut werden wo die Gülle zur Verfügung steht oder wo sich ein Wärmeabnehmer befindet.

Ab einer elektrischen Leistung der Biogasanlage von 400 bis 500 kW, je nach Energiedichte bzw. Tagesration der organischen Rohstoffe, ist es sinnvoll einen zweiten Hydrolysebehälter 50 (in der Zeichnung angedeutet) zu installieren. Der zweite Hydrolysebehälter 50 ist durch eine Nachspeiseleitung 52 mit dem ersten Behälter 10 verbunden. In der Nachspeiseleitung 52 angeordnet sind eine Nachspeisepumpe 51 und ein Durchflussmesser 53. Die Fütterung des Fermenters erfolgt, bei vorhandenem zweitem Hydrolysebehälter 50, immer aus dem zweiten Hydrolysebehälter 50, über eine Absaugleitung 54. In diesem zweiten Hydrolysebehälter 50 läuft der Versäuerungsprozess nahezu schwankungsfrei ab, zumal er nicht direkt gefüttert wird.

Die Absaugleitung 54 verbindet den zweiten Hydrolysebehälter 50 mit der Pumpe 28. In der Absaugleitung 54 ist ein automatischer Schieber angeordnet, der den zweiten Hydrolysebehälter 50 von der Pumpe 28 trennt. Die gleiche Substratmenge, die über die Absaugleitung 54 zum Fermenter gefördert wird, wird über die Nachspeiseleitung 52 aus dem Behälter 10 in den zweiten Hydrolysebehälter 50 nachgefördert. Denkbar ist es aber auch, die Nachspeiseleitung 52 als freien Überlauf vom Behälter 10 zum zweiten Hydrolysebehälter 50 auszuführen. Die Zielsetzung dabei ist, einen möglichst hohen gleichbleibenden Betriebsfüllstand im zweiten Hydrolysebehälter 50 zu halten.

Eine zweite drehzahlgeregelte Pumpe 56 saugt Substrat aus dem zweiten Hydrolysebehälter 50 und speist sie über eine Druckleitung 58, stromabwärts der ersten frequenzgeregelten Pumpe 35, in die Zulaufleitung 34 ein. Durch Mischung von Substrat und Verdünnungsflüssigkeit in der Zulaufleitung 34 sinkt der pH-Wert der Verdünnungsflüssigkeit vor Eintritt in den Behälter 10 ab, so dass Methanausgasungen im Behälter 10 weitestgehend vermieden werden. Sowohl in der Überlaufleitung 52 als auch in der Absaugleitung 54 sind Schieber 60 angeordnet, so dass der zweite Hydrolysebehälter 50 durch Öffnen der Schieber in die Biogasanlage eingekoppelt wird. Beziehungsweise, es können damit die beiden Behälter 10 oder 50 bei Reparatur- oder Reinigungsarbeiten auch einzeln betrieben werden. Dafür weist der zweite Hydrolysebehälter 50 eine Einfüllöffnung auf.

Die Funktionsweise des Behälters 10 ist folgendermaßen: Um ein rühr- und pumpfähiges Substrat zu erhalten, wird Verdünnungsflüssigkeit in den Behälter 10 gefördert. Dies kann wie zu Beginn beschrieben, kontinuierlich mehrmals stündlich in kleinen Mengen erfolgen, um einen gleichbleibenden Füllstand zu erreichen. Die Menge an Verdünnungsflüssigkeit entspricht der 3 bis 4-fachen Menge an organischen Feststoffen. Die Verdünnungsflüssigkeit umfasst beispielsweise flüssige Gärreste, die mit der frequenzgeregelten Pumpe 35 aus einem Gärrestelager (nicht dargestellt) in den Behälter 10 gepumpt werden. Wird die Verdünnungsflüssigkeit in der Zulaufleitung 34 mittels der thermischen Aufbereitung 38 auf über 60°C erhitzt, sinkt der pH-Wert der Verdünnungsflüssigkeit etwas ab und die darin enthaltenen methanogenen Bakterien werden durch Wärmeeinwirkung abgetötet. Dadurch wird eine Methanausgasung im Behälter 10 reduziert.

Gleichzeitig wurde bei einer Praxisanlage in der so behandelten Verdünnungsflüssigkeit aus dem Nachgärer ein ca. 5-fach höherer Säuregehalt (ca. 500 mg/l anstelle 100 mg/l) in der unbehandelten Verdünnungsflüssigkeit gemessen. Die zusätzlich gelösten Säuren (hauptsächlich Essigsäure) können im Fermenter zur Methanerzeugung genutzt werden, woraus sich eine höhere Gasausbeute aus den eingesetzten Rohstoffen ergibt.

Die thermische Aufbereitung 38 umfasst beispielsweise einen druckgeprüften Durchfluss-Wärmeübertrager. Die Erhitzung der Verdünnungsflüssigkeit erfolgt dabei im Gleichstrom oder im Gegenstrom mit Warmwasser. Ein Teilstrom der Verdünnungsflüssigkeit wird dabei von einem Austritt des Durchfluss-Wärmeübertragers über eine in der Figur nicht dargestellte Bypassleitung, die mittels einer, als Schieber ausgeführten, Absperrarmatur, die stufenlose verschlossen werden kann, auf die Saugseite der Pumpe 35 zurückgeführt. Damit wird ein Unterschreiten einer Mindesttemperatur von circa 63 °C im Durchfluss-Wärmeübertrager vermieden. Wird die Mindesttemperatur unterschritten, wird die Pumpe 35 abgeschaltet. Somit wird durch die voranstehend beschriebene Rückführung eines Teilstroms der Verdünnungsflüssigkeit ein häufiges Ab- und Anschalten der Pumpe 35 verhindert. Durch die thermische Aufbereitung 38, insbesonders bei Temperaturen >70°C, ist mit einer etwas erhöhten Gasausbeute zu rechnen.

Mit dem vorgeschalteten Durchfluss-Wärmeübertrager könnte auch eine Vorkühlung der Verdünnungsflüssigkeit auf Temperaturen kleiner etwa 32° bzw. 35° vorgenommen werden, da ab diesem Temperaturbereich die methanogenen Bakterien inaktiv werden und so ebenfalls eine Methanausgasung in der Hydrolyse, wenn diese unter ca. 32° betrieben wird, verhindert werden. Hierzu kann das in der Wandheizung befindliche Wasser mit Hilfe eines Kühlaggregates heruntergekühlt werden. Um die Kühlleistung deutlich zu erhöhen wird der Durchfluss-Wärmeübertrager ebenfalls mit kaltem Wasser betrieben.

Optional kann über die Belüftungseinrichtung 40 ein Sauerstoffgehalt der Verdünnungsflüssigkeit erhöht werden, wodurch die methanogenen Bakterien gehemmt werden.

Über den Behältereinfüllstutzen 42 strömt die Verdünnungsflüssigkeit in den Behälter 10. Weil der Behältereinfüllstutzen 42 als 90°-Bogen ausgeführt ist, strömt die Verdünnungsflüssigkeit an der Behälterwand 44 entlang. Durch die zylindrische Form der Behälterwand 44 wird die einströmende Verdünnungsflüssigkeit auf eine Kreisbahn gezwungen. Die im Einströmbereich des Behältereinfüllstutzens angeordnete pH-Sonde wird von der einströmenden Verdünnungsflüssigkeit sofort umspült, so dass die pH-Sonde stets den pH-Wert der frisch eingeströmten Verdünnungsflüssigkeit, gemischt mit dem Behälterinhalt, misst. Der horizontale Abstand der, im Einströmbereich des Behältereinfüllstutzens angeordneten, pH-Sonde zum Einfüllstutzen 42 beträgt ca. 1 m.

Solange der gemessene pH-Wert unterhalb eines vorgebbaren Schwellwertes liegt, wird weiter Verdünnungsflüssigkeit in den Behälter 10 gefördert.

Um den pH-Wert in der einströmenden Verdünnungsflüssigkeit zu reduzieren, werden verschiedene Maßnahmen angewendet. Zunächst kann, wie bereits voranstehend beschrieben, die Verdünnungsflüssigkeit erhitzt werden. Als weitere Maßnahme wird die Verdünnungsflüssigkeit mit vergleichsweise saurem (niedriger pH-Wert) Substrat aus dem Behälter 10 gemischt. Dazu läuft die Pumpe 28 an und der automatische Schieber 33 wird geöffnet. Gleichzeitig ist der automatische Schieber 31 geschlossen. Die Pumpe 28 saugt flüssiges Substrat mit niedrigem pH-Wert aus dem Behälter 10 an und fördert es über die Bypassleitung 32 in die Zulaufleitung 34, wo es sich mit der Verdünnungsflüssigkeit vermischt und so der pH-Wert der einströmenden Flüssigkeit absinkt.

Überschreitet der pH-Wert an der pH-Sonde trotz der vorangehend beschriebenen Maßnahmen einen vorgegebenen Schwellwert, wird die Zufuhr von Verdünnungsflüssigkeit unterbrochen, bis der pH-Wert einen bestimmten Wert unterschreitet. Dadurch Verlängert sich die Zeit, die benötigt wird, um die gewünschte Menge an Verdünnungsflüssigkeit dem Behälter 10 zuzuführen. Weil ein Einbringen der festen organischen Stoffe erst im Anschluss erfolgen kann, verringert sich die sogenannte Energiedichte des Behälters 10.

Um Auswirkungen auf die Biogasproduktion im Fermenter zu verhindern, beinhaltet das erfindungsgemäße Verfahren eine Auslastungsoptimierungsfunktion. Dabei wird von der Anlagensteuerung ein Gasspeicherfüllstand überwacht. Fällt der Gasspeicherfüllstand unter einen vorgebbaren Wert, wird eine Fütterungsmenge an Substrat in den Fermenter erhöht, bis ein oberer Gasspeicherfüllstand erreicht wird. Einer Gasüberproduktion wird entgegengewirkt, indem ein Maximalwert für den Gasspeicherfüllstand festgelegt wird. Wird dieser Maximalwert überschritten, wird die Fütterungsmenge um einen vorgegeben Wert (prozentual) reduziert, bis ein festgelegter Wert erreicht wird. Dann wird wieder die normale Fütterungsmenge eingestellt.

Die Auslastungsoptimierungsfunktion, sowohl bei Gasüberproduktion als auch bei Gasunterproduktion, wird aus sicherheitstechnischen Gründen nur nach manueller Freigabe durch den Anlagenfahrer aktiviert. Da bei einem bereits überlasteten Fermenter die Futterration nicht automatisch erhöht werden soll.

Optional kann auch ein Methangehalt-Grenzwert eingegeben werden, bei dessen Unterschreitung die Auslastungsoptimierungsfunktion automatisch abschaltet, bzw. gar nicht aktiviert wird. Beim Einsatz eines variabel zuschaltbaren zweiten Motors sind große Steigerungen in kurzer Zeit möglich. Um auf diese Situation schnell reagieren zu können, ist die Eingabe einer zweiten Futterrationserhöhung bzw. Reduzierung möglich. Hintergrund sind die höheren Vergütungssätze für bedarfsgerechte Stromerzeugung.

Die Eingabe der Werte und die Freigabe der Auslastungsoptimierungsfunktion erfolgt über eine Bildschirmmaske dargestellt in Figur 2.

Alle Parameter des Hydrolyseverfahrens, insbesonders der pH-Wert, Temperatur, Laufzeiten der frequenzgeregelten Pumpen 35, 56 sowie die damit zugeführte Menge an Verdünnungsflüssigkeit, Füllstand, Rührzeiten, Gasfüllstand im Fermenter, Einstellwerte der Auslastungsoptimierung, Motorleistung, Eigenenergiebedarf, Methangehalt im Biogas und Tagesmenge der zugeführten organischen Stoffe, aufgesplittet nach Art der Stoffe, werden von der Anlagensteuerung in einem Logbuch gespeichert. In einer Praxisanlage werden zur Zeit stündlich 55 Werte aufgezeichnet. Der Betreiber kann sich ein Tageslogbuch ausdrucken mit 55 Spaltenwerten pro Stundenzeile (24 / Tag). In Zeile 25 können die Summen oder Durchschnittswerte der einzelnen Spalten gebildet werden. Diese Summenzeile kann ebenfalls gespeichert und zur Optimierung der Einstellparameter bzw. für statistische Zwecke genutzt werden. Der Verlauf der Parameter über die Zeit, wird mittels verschiedenfarbiger Graphen dargestellt.

Die im Logbuch gespeicherten Daten werden mit einem Algorithmus ausgewertet. Der Algorithmus ermittelt eine Versäuerungskapazität genannte Kennzahl. Die Versäuerungskapazität stellt einen Volumenstrom an Verdünnungsflüssigkeit in m³/h dar, die dem Behälter 10, unter Einhaltung eines vorgegeben pH-Wertes, zugeben werden kann. Alle erreichten maximalen Versäuerungskapazitäten während einer festgelegten Zeitspanne werden aufgezeichnet. In einem nächsten Schritt schlägt die Anlagensteuerung dem Anlagenfahrer, gegebenenfalls in Abhängigkeit der Art der organischen Stoffe, die optimalen Parameter für den Hydrolyseprozess vor.

So kann beispielsweise bei Einbringung von organischen Stoffen, die über ein hohe Pufferkapazität verfügen und die eine pH-Wert Erhöhung bewirken, wie Rindermist oder Hühnertrockenkot, kurzfristig die Hydrolysetemperatur erhöht werden um die Versäuerung zu beschleunigen. Ist diese Funktion vom Anlagenfahrer freigegeben, erfolgt die Temperaturerhöhung automatisch bei Beginn der Fütterung der organischen Stoffe. Gleiches gilt auch für die Laufzeiten der Rührwerke 14 bzw. 22.Diese kann in dieser Prozessphase, nach Freigabe durch den Anlagenfahrer, deutlich verlängert werden, um eine schnellere Versäuerung zu erreichen.

## Patentansprüche

1. Verfahren zum Betrieb eines Behälters (10) zur Vorbehandlung organischer Stoffe in einer Biogasanlage, **dadurch gekennzeichnet, dass** dem Behälter (10) Verdünnungsflüssigkeit zugeführt wird solange ein vorgegebener pH-Wert und/oder Betriebsfüllstand nicht überschritten ist.

2. Verfahren nach vorangehendem Anspruch 1, **dadurch gekennzeichnet, dass** die Verdünnungsflüssigkeit je nach Erfordernis erhitzt oder gekühlt wird.

3. Verfahren nach einem der vorangehenden Ansprüche, **dadurch gekennzeichnet, dass** eine Temperatur im Behälter (10) in Abhängigkeit des pH-Werts gesteuert wird.

4. Verfahren nach einem der vorangehenden Ansprüche, **dadurch gekennzeichnet, dass** die Verdünnungsflüssigkeit im Behälter 10 oder bereits in einer Zufuhrleitung (34) mit Substrat gemischt wird.

5. Verfahren nach einem der vorangehenden Ansprüche, **dadurch gekennzeichnet, dass** bereits versäuertes Substrat aus einem zweiten Hydrolysebehälter (50) dem Behälter (10) zugeführt wird.

6. Verfahren nach einem der vorangehenden Ansprüche, **dadurch gekennzeichnet, dass** bei einem sinkenden Gasspeicherfüllstand eine Fütterungsmenge um einen vorgegeben Wert erhöht wird und optionaler Eingabemöglichkeit eines zweiten Wertes für Zuschaltung eines weiteren Motors.

7. Verfahren nach einem der vorangehenden Ansprüche, **dadurch gekennzeichnet, dass** ab einem vorgegeben maximalen Gasspeicherfüllstand die Fütterungsmenge um einen vorgegebenen Wert reduziert wird und optionaler Eingabemöglichkeit eines zweiten Wertes für Zuschaltung eines weiteren Motors.

8. Verfahren nach einem der vorangehenden Ansprüche, **dadurch gekennzeichnet, dass** eine automatische Änderung der Fütterungsmenge von einem Anwender freigeschaltet werden muss.

9. Verfahren nach einem der vorangehenden Ansprüche, **dadurch gekennzeichnet, dass** eine Versäuerungskapazität ermittelt wird, wobei die Versäuerungskapazität denjenigen Volumenstrom an Verdünnungsflüssigkeit in m³/h angibt, der dem Behälter 10 unter Einhaltung vorgegebener pH-Wert Grenzen zugeführt werden kann.

10. Verfahren nach einem der vorangehenden Ansprüche, **dadurch gekennzeichnet, dass** Parameter des Verfahrens, insbesondere die Versäuerungskapazität - bevorzugt in einem Logbuch - aufgezeichnet und ausgewertet werden und dass, basieren auf der Auswertung, dem Anwender Parameter vorgeschlagen werden.

11. Verfahren nach einem der vorangehenden Ansprüche, **dadurch gekennzeichnet, dass** der Füllstand des Hydrolysebehälters (10) auf einen vorgebbaren Betriebsfüllstand geregelt wird.

12. Verfahren nach einem der vorangehenden Ansprüche, **dadurch gekennzeichnet, dass** die Feststoff-Fütterung mindestens an einem Wochentag unterbrochen wird bei gleichzeitigem, gleichbleibenden Weiterbetrieb der Biogas-Anlage.

13. Verfahren nach einem der vorhergehenden Ansprüche, **dadurch gekennzeichnet, dass** die Verdünnungsflüssigkeit auf Temperaturen kleiner 35° C, bevorzugt kleiner 32° C gekühlt wird.

14. Verfahren nach einem der vorhergehenden Ansprüche, **dadurch gekennzeichnet, dass** in einer zentralen Hydrolyse-Station (10) eine Hydrolyse-Energiebrei (HEB) erzeugt und mittels Tankfahrzeug an mindestens eine Satelliten-Biogas-Anlagen befördert wird.

15. Verfahren nach einem der vorhergehenden Ansprüche, **dadurch gekennzeichnet, dass** die Verdünnungsflüssigkeit thermisch behandelt, insbesondere gekühlt oder vorgewärmt wird.

16. Behälter (10) zur Vorbehandlung organischer Stoffe einer Biogasanlage umfassend eine Behältereinlaufstutzen (42) und eine pH-Sonde (48), **dadurch gekennzeichnet, dass** der Behältereinlaufstutzen (42) als 90°-Bogen ausgeführt ist, wobei eine, durch den Behältereinlaufstutzen (42) in den Behälter (10) einströmende Verdünnungsflüssigkeit die pH-Sonde (48) umströmt.

17. Behälter (10) nach Anspruch 16, **dadurch gekennzeichnet, dass** ein Intensivzerkleinerer (70) vorgesehen ist.

18. Steuergerät zum Betreiben einer Biogasanlage, **dadurch gekennzeichnet, dass** es die Durchführung des Verfahrens nach vorangehenden Ansprüchen 1 bis 15 ermöglicht.

19. Computerprogramm für ein Steuergerät zum Betreiben eine Biogasanlage, **dadurch gekennzeichnet, dass** es nach dem Verfahren nach vorhergehenden Ansprüchen 1 bis 15 arbeitet, wenn es auf einem Computer oder einem Steuergerät abläuft.

20. Computerprogramm nach Anspruch 19, **dadurch gekennzeichnet, dass** es auf einem digitalen Speichermedium abgespeichert ist.
